# EUROPEAN PATENT APPLICATION

(11) **EP 4 830 825 A1**
(43) Date of publication of application: **28.10.2026**
(21) Application number: 23962033.9
(22) Date of filing: 23.12.2023
(51) Int. Cl.: B01J 8/24

(54) **HIGH-DENSITY FAST FLUIDIZED BED REACTOR AND USE METHOD THEREFOR**

(30) Priority: 19.12.2023 CN 202311753070
(71) Applicant: Dalian Institute Of Chemical Physics, Chinese Academy Of Sciences, Liaoning 116023 (CN)
(72) Inventor: YE, Mao, Dalian, Liaoning 116023 (CN); ZHANG, Tao, Dalian, Liaoning 116023 (CN); ZHANG, Jinling, Dalian, Liaoning 116023 (CN); TANG, Hailong, Dalian, Liaoning 116023 (CN); WANG, Jing, Dalian, Liaoning 116023 (CN); ZHANG, Cheng, Dalian, Liaoning 116023 (CN); JIA, Jinming, Dalian, Liaoning 116023 (CN); MA, Xiangang, Dalian, Liaoning 116023 (CN); WANG, Xiangao, Dalian, Liaoning 116023 (CN); XU, Shuliang, Dalian, Liaoning 116023 (CN); LIU, Zhongmin, Dalian, Liaoning 116023 (CN)
(74) Representative: Barker Brettell LLP
(86) International application number: PCT/CN2023/141300
(87) International publication number: WO 2025/129711

(57) **Abstract**

A high-density fast fluidized bed reactor and a use method therefor. The high-density fast fluidized bed reactor comprises: a reactor outer shell (1), a reactor inner shell (2), a delivery pipe (3), a raw material distributor (4), a first gas-solid separation device (5), a gas collection chamber (6), fluidizing steam distributor (7), catalyst distribution pipe (8), a second gas-solid separation device (9), a product gas delivery pipe (10), a catalyst input pipe (11) and a catalyst withdrawal pipe (12). The main functional areas of the reactor are a reaction zone (A), a catalyst retention zone (B) and a gas-solid separation zone (C). The bottom of the reaction zone (A) is in communication with the bottom of the catalyst retention zone (B), and the circulation intensity of catalyst flowing from the catalyst retention zone (B) to the reaction zone (A) is as high as 500-1000kg/(m²·s), thereby achieving high bed density in the reaction zone (A) under the condition of high superficial linear velocity.

## Description

### TECHNICAL FIELD

The present application relates to a high-density fast fluidized bed reactor and a use method therefor, belonging to the field of chemical catalysis.

### BACKGROUND

Ethylene, propylene and other low-carbon olefins are important basic organic chemical raw materials and the cornerstone of modern chemical industry. Traditional production technologies are highly dependent on petroleum resources. Therefore, utilizing China's relatively abundant coal resources to replace petroleum resources has important strategic significance.

Olefin production technology routes mainly include naphtha cracking to olefins, methanol to olefins, propane dehydrogenation to propylene, and alkane cracking to olefins. Low-carbon olefins are very active and prone to polymerization, alkylation, aromatization and other reactions to generate by-products, thereby reducing the yield of low-carbon olefins.

Methanol to olefins (MTO) technology targets ethylene and propylene as products. Methanol is converted under the action of molecular sieve catalysts into a mixture containing ethylene, propylene, butene, pentene, alkanes and other components. In 2010, the Shenhua Baotou methanol to olefins plant using the DMTO technology developed by the Dalian Institute of Chemical Physics, Chinese Academy of Sciences, was completed and put into operation. This was the first global industrial application of MTO technology. As of 2023, 16 DMTO industrial units have been put into operation, with a total low-carbon olefin production capacity of approximately 9.3 million tons/year. Taking DMTO technology as an example, the carbon-based selectivity of ethylene and propylene is approximately 80%wt, and the selectivity of butene and pentene is approximately 15 %wt.

Improving the yield of ethylene and propylene is a major goal of olefin production technology advancement.

### SUMMARY

Methanol to olefins technology uses SAPO molecular sieve catalysts. The hydrocarbon pool mechanism holds that methanol is converted into ethylene, propylene and other products through aromatic cycles or olefin cycles in molecular sieve catalysts. The main reactions include:

CH₃OH → C₂H₄ + C₃H₆ (1)

C₂H₄ + CH₃OH → C₃H₆ (2)

C₃H₆ + CH₃OH → C₄H₈ (3)

C₄H₈ + CH₃OH → C₅H₁₀ (4)

Reactions (1) and (2) generate ethylene and propylene. Reactions (2), (3) and (4) show that small molecule olefins are relatively active and are further converted into large molecule olefins, thereby reducing the yield of ethylene and propylene.

Methanol to olefins technology uses fluidized bed reactors. Increasing the capacity of a single reactor and inhibiting the conversion of small molecule olefins to large molecule olefins are the core objectives of developing methanol to olefins reactors. An effective method to increase the capacity of a single reactor is to increase the superficial linear velocity of the fluidized bed reactor to achieve the purpose of increasing raw material feed rate. In addition, increasing the superficial linear velocity of the fluidized bed reactor and reducing the residence time of product gas in the reactor can effectively inhibit the generation of large molecule olefins. However, increasing the superficial linear velocity usually causes the bed density of the reaction zone of the fluidized bed reactor to decrease significantly, and the catalyst inventory also decreases significantly, leading to a decrease in methanol conversion rate. That is, there is a negative correlation between raw material feed rate and bed density of the fluidized bed reaction zone.

To solve the above technical problems, the present application discloses a high-density fast fluidized bed reactor that achieves high bed density under high superficial linear velocity conditions, improves raw material conversion rate, and simultaneously inhibits side reactions such as polymerization, alkylation and aromatization of small molecule olefins.

According to a first aspect of the present application, a high-density fast fluidized bed reactor is provided.

A high-density fast fluidized bed reactor, wherein the high-density fast fluidized bed reactor comprises a reactor outer shell, a reactor inner shell, a delivery pipe, a first gas-solid separation device, and a second gas-solid separation device;
The reactor inner shell is located in a lower part of the reactor outer shell;
The delivery pipe is located in a central region of an upper-middle part of the high-density fast fluidized bed reactor;
A region enclosed by the reactor inner shell is a reaction zone;
A bottom end of the delivery pipe is in communication with a top end of the reaction zone;
An annular region enclosed by the reactor outer shell and the reactor inner shell is a catalyst retention zone;
A bottom of the reaction zone is in communication with a bottom of the catalyst retention zone;
A region enclosed by the reactor outer shell and the delivery pipe is a gas-solid separation zone;
The catalyst retention zone is in communication with the gas-solid separation zone and is located below the gas-solid separation zone;
The first gas-solid separation device and the second gas-solid separation device are both located in the gas-solid separation zone;
An upper opening of the delivery pipe is connected to an inlet of the first gas-solid separation device;
A catalyst outlet of the first gas-solid separation device is located in a lower part of the gas-solid separation zone.

Optionally, an inlet of the second gas-solid separation device is located in the gas-solid separation zone, and a catalyst outlet of the second gas-solid separation device is located in a lower part of the gas-solid separation zone.

Optionally, the first gas-solid separation device and the second gas-solid separation device each independently adopt one or more groups of gas-solid cyclone separators, each group of gas-solid cyclone separators comprising a first-stage gas-solid cyclone separator and a second-stage gas-solid cyclone separator.

Optionally, the high-density fast fluidized bed reactor comprises a reactor gas collection chamber and a product gas delivery pipe;
The reactor gas collection chamber is located at a top of the high-density fast fluidized bed reactor;
The product gas delivery pipe is connected to a top of the reactor gas collection chamber;
A gas outlet of the first gas-solid separation device and a gas outlet of the second gas-solid separation device are both connected to the reactor gas collection chamber.

Optionally, the high-density fast fluidized bed reactor comprises a catalyst distribution pipe, a fluidizing steam distributor, a raw material distributor, a catalyst withdrawal pipe, and a catalyst input pipe;
The catalyst distribution pipe passes through the reactor inner shell to communicate the catalyst retention zone and the reaction zone;
The fluidizing steam distributor is located at a bottom of the catalyst retention zone;
The raw material distributor is located at a bottom of the reaction zone;
The catalyst withdrawal pipe passes through the reactor outer shell and is located in a lower part of the catalyst retention zone;
The catalyst input pipe passes through the reactor outer shell and is located in a lower part of the gas-solid separation zone.

Optionally, a lower surface of the catalyst distribution pipe has openings.

As a preferred embodiment, the high-density fast fluidized bed reactor comprises: a reactor outer shell (1), a reactor inner shell (2), a delivery pipe (3), a raw material distributor (4), a first gas-solid separation device (5), a gas collection chamber (6), a fluidizing steam distributor (7), a catalyst distribution pipe (8), a second gas-solid separation device (9), a product gas delivery pipe (10), a catalyst input pipe (11), and a catalyst withdrawal pipe (12).

The region enclosed by the reactor inner shell is the reaction zone, the annular region enclosed by the reactor outer shell and the reactor inner shell is the catalyst retention zone, the bottom of the reaction zone is in communication with the bottom of the catalyst retention zone, the region enclosed by the reactor outer shell and the delivery pipe is the gas-solid separation zone, and the catalyst retention zone is in communication with the gas-solid separation zone and is located below the gas-solid separation zone.

The raw material distributor is located at the bottom of the reaction zone, the delivery pipe is located in the central region of the upper-middle part of the high-density fast fluidized bed reactor, the bottom end of the delivery pipe is connected to the top end of the reaction zone, and the upper part of the delivery pipe is connected to the inlet of the first gas-solid separation device.

The first gas-solid separation device is located in the gas-solid separation zone, the gas outlet of the first gas-solid separation device is connected to the gas collection chamber, and the catalyst outlet of the first gas-solid separation device is located in the lower part of the gas-solid separation zone.

The fluidizing steam distributor is located at the bottom of the catalyst retention zone.

The catalyst distribution pipe passes through the reactor inner shell to communicate the catalyst retention zone and the reaction zone, and the lower surface of the catalyst distribution pipe has openings.

The second gas-solid separation device is located in the gas-solid separation zone, the inlet of the second gas-solid separation device is located in the gas-solid separation zone, the gas outlet of the second gas-solid separation device is connected to the gas collection chamber, and the catalyst outlet of the second gas-solid separation device is located in the lower part of the gas-solid separation zone.

The gas collection chamber is located at the top of the high-density fast fluidized bed reactor, and the product gas delivery pipe is connected to the top of the gas collection chamber.

The catalyst input pipe passes through the reactor outer shell and is located in the lower part of the gas-solid separation zone.

The catalyst withdrawal pipe passes through the reactor outer shell and is located in the lower part of the catalyst retention zone.

According to a second aspect of the present application, a use method of the above-mentioned high-density fast fluidized bed reactor is provided.

The use method of the above-mentioned high-density fast fluidized bed reactor, wherein the method comprises:
(S-1) Catalyst enters the gas-solid separation zone through the catalyst input pipe, and then enters the catalyst retention zone;
(S-2) Vaporized raw material enters the reaction zone from the raw material distributor, contacts the catalyst, and generates product gas. The product gas carries the catalyst through the delivery pipe into the first gas-solid separation device. After gas-solid separation, the catalyst enters the catalyst retention zone;
(S-3) Steam enters the catalyst retention zone from the fluidizing steam distributor. The steam carries a small amount of catalyst from the catalyst retention zone into the second gas-solid separation device in the gas-solid separation zone. After gas-solid separation, the catalyst returns to the catalyst retention zone;
(S-4) Catalyst in the catalyst retention zone enters the reaction zone through the catalyst distribution pipe; catalyst in the catalyst retention zone enters the bottom of the reaction zone through the bottom of the catalyst retention zone;
(S-5) Catalyst in the catalyst retention zone is discharged through the catalyst withdrawal pipe.

Optionally, the product gas in step (S-2) and the steam in step (S-3) enter the reactor gas collection chamber and enter the downstream process section through the product gas delivery pipe.

Optionally, the process operating conditions of the reaction zone are: gas superficial linear velocity of 1.5-7.0m/s, temperature of 350-700°C, pressure of 50-500kPa, bed density of 100-500kg/m³, and reaction contact time of 0.5~4s.

Optionally, the process operating conditions of the catalyst retention zone are: gas superficial linear velocity of 0.02-0.2m/s, temperature of 350-700°C, and bed density of 500-900kg/m³.

Optionally, the circulation intensity of catalyst flowing from the catalyst retention zone to the reaction zone is 500-1000kg/(m²·s).

Optionally, the product gas is a product gas containing olefins.

Optionally, the raw material comprises methanol and/or dimethyl ether.

When the raw material comprises methanol and/or dimethyl ether, the catalyst is a SAPO molecular sieve.

Optionally, the SAPO molecular sieve is selected from SAPO-34 and/or SAPO-18 molecular sieves.

Optionally, the raw material comprises C₃+ hydrocarbons.

Specifically, the raw material is mixed C4 or naphtha.

When the raw material comprises C₃+ hydrocarbons, the catalyst is ZSM-5 molecular sieve.

Wherein C₃+ hydrocarbons refer to hydrocarbons with a carbon atom number greater than or equal to 3 in the molecule.

As a preferred embodiment, the use method of the high-density fast fluidized bed reactor comprises:
a. Catalyst enters the gas-solid separation zone through the catalyst input pipe, and then enters the catalyst retention zone;
b. Vaporized raw material enters the reaction zone from the raw material distributor, contacts the catalyst, and generates product gas containing olefins. The product gas carries the catalyst through the delivery pipe into the first gas-solid separation device. After gas-solid separation, the product gas enters the gas collection chamber, and the catalyst enters the catalyst retention zone;
c. Steam enters the catalyst retention zone from the fluidizing steam distributor. The steam carries a small amount of catalyst from the catalyst retention zone into the second gas-solid separation device in the gas-solid separation zone. After gas-solid separation, the steam enters the gas collection chamber, and the catalyst returns to the catalyst retention zone;
d. Product gas and steam enter the downstream process section through the product gas delivery pipe;
f. Catalyst in the catalyst retention zone enters the reaction zone through the catalyst distribution pipe; catalyst in the catalyst retention zone enters the bottom of the reaction zone through the bottom of the catalyst retention zone;
g. Catalyst in the catalyst retention zone is discharged through the catalyst withdrawal pipe.

The beneficial effects that can be produced by the present application include:
(1) The high-density fast fluidized bed reactor provided by the present application mainly comprises a reaction zone, a catalyst retention zone and a gas-solid separation zone. The circulation intensity of catalyst flowing from the catalyst retention zone to the reaction zone is as high as 500-1000kg/(m²·s), thereby increasing the bed density of the reaction zone, achieving high bed density under high superficial linear velocity conditions, and overcoming the negative correlation between raw material feed rate and bed density of the fluidized bed reaction zone.
(2) The high-density fast fluidized bed reactor provided by the present application adopts a structure in which the first gas-solid separation device is directly connected to the delivery pipe, achieving rapid separation of product gas and catalyst, significantly shortening the gas-solid contact time, and inhibiting side reactions.
(3) The high-density fast fluidized bed reactor provided by the present application is suitable for molecular sieve catalytic reaction processes targeting small molecule olefins as products.

### BRIEF DESCRIPTION OF THE DRAWINGS

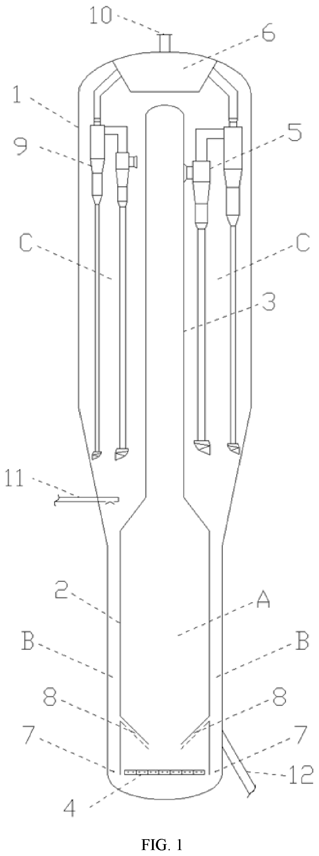

FIG. 1 is a schematic diagram of a high-density fast fluidized bed reactor according to one embodiment of the present application.

The reference numerals in FIG. 1 are explained as follows:
1 - reactor outer shell
2 - reactor inner shell
3 - delivery pipe
4 - raw material distributor
5 - first gas-solid separation device
6 - gas collection chamber
7 - fluidizing steam distributor
8 - catalyst distribution pipe
9 - second gas-solid separation device
10 - product gas delivery pipe
11 - catalyst input pipe
12 - catalyst withdrawal pipe

### DETAILED DESCRIPTION OF THE EMBODIMENTS

The present application is described in detail below in conjunction with embodiments, but the present application is not limited to these embodiments.

Unless otherwise specified, the raw materials and catalysts in the embodiments of the present application are all purchased through commercial channels.

In one specific embodiment, the schematic diagram of the high-density fast fluidized bed reactor described in the present application is shown in FIG. 1, mainly comprising a reaction zone (A), a catalyst retention zone (B) and a gas-solid separation zone (C), described separately as follows:
1. The high-density fast fluidized bed reactor comprises: a reactor outer shell (1), a reactor inner shell (2), a delivery pipe (3), a raw material distributor (4), a first gas-solid separation device (5), a gas collection chamber (6), a fluidizing steam distributor (7), a catalyst distribution pipe (8), a second gas-solid separation device (9), a product gas delivery pipe (10), a catalyst input pipe (11), and a catalyst withdrawal pipe (12); the region enclosed by the reactor inner shell (2) is the reaction zone (A), the annular region enclosed by the reactor outer shell (1) and the reactor inner shell (2) is the catalyst retention zone (B), the bottom of the reaction zone (A) is in communication with the bottom of the catalyst retention zone (B), the region enclosed by the reactor outer shell (1) and the delivery pipe (3) is the gas-solid separation zone (C), and the catalyst retention zone (B) is in communication with the gas-solid separation zone (C) and is located below the gas-solid separation zone (C);
2. The raw material distributor (4) is located at the bottom of the reaction zone (A), the delivery pipe (3) is located in the central region of the upper-middle part of the high-density fast fluidized bed reactor, the bottom end of the delivery pipe (3) is connected to the top end of the reaction zone (A), and the upper part of the delivery pipe (3) is connected to the inlet of the first gas-solid separation device (5); the first gas-solid separation device (5) is located in the gas-solid separation zone (C), the gas outlet of the first gas-solid separation device (5) is connected to the gas collection chamber (6), and the catalyst outlet of the first gas-solid separation device (5) is located in the lower part of the gas-solid separation zone (C); the fluidizing steam distributor (7) is located at the bottom of the catalyst retention zone (B); the catalyst distribution pipe (8) passes through the reactor inner shell (2) to communicate the catalyst retention zone (B) and the reaction zone (A), and the lower surface of the catalyst distribution pipe (8) has openings; the second gas-solid separation device (9) is located in the gas-solid separation zone (C), the inlet of the second gas-solid separation device (9) is located in the gas-solid separation zone (C), the gas outlet of the second gas-solid separation device (9) is connected to the gas collection chamber (6), and the catalyst outlet of the second gas-solid separation device (9) is located in the lower part of the gas-solid separation zone (C); the gas collection chamber (6) is located at the top of the high-density fast fluidized bed reactor, the product gas delivery pipe (10) is connected to the top of the gas collection chamber (6), the catalyst input pipe (11) passes through the reactor outer shell (1) and is located in the lower part of the gas-solid separation zone (C); the catalyst withdrawal pipe (12) passes through the reactor outer shell (1) and is located in the lower part of the catalyst retention zone (B).

Specifically, the first gas-solid separation device (5) adopts multiple groups of gas-solid cyclone separators, each group of gas-solid cyclone separators comprising a first-stage gas-solid cyclone separator and a second-stage gas-solid cyclone separator.

Specifically, the second gas-solid separation device (9) adopts multiple groups of gas-solid cyclone separators, each group of gas-solid cyclone separators comprising a first-stage gas-solid cyclone separator and a second-stage gas-solid cyclone separator.

In one specific embodiment, the use method of the high-density fast fluidized bed reactor described in the present application comprises the following steps:
a. Catalyst enters the gas-solid separation zone (C) through the catalyst input pipe (11), and then enters the catalyst retention zone (B);
b. Vaporized raw material enters the reaction zone (A) from the raw material distributor (4), contacts the catalyst, and generates product gas containing olefins. The product gas carries the catalyst through the delivery pipe (3) into the first gas-solid separation device (5). After gas-solid separation, the product gas enters the gas collection chamber (6), and the catalyst enters the catalyst retention zone (B);
c. Steam enters the catalyst retention zone (B) from the fluidizing steam distributor (7). The steam carries a small amount of catalyst from the catalyst retention zone (B) into the second gas-solid separation device (9) in the gas-solid separation zone (C). After gas-solid separation, the steam enters the gas collection chamber (6), and the catalyst returns to the catalyst retention zone (B);
d. Product gas and steam enter the downstream process section through the product gas delivery pipe (10);
e. Catalyst in the catalyst retention zone (B) enters the reaction zone (A) through the catalyst distribution pipe (8); catalyst in the catalyst retention zone (B) enters the bottom of the reaction zone (A) through the bottom of the catalyst retention zone (B);
f. Catalyst in the catalyst retention zone (B) is discharged through the catalyst withdrawal pipe (12).

In the method described in the present application, the calculation methods for raw material single-pass conversion rate and product selectivity are as follows: 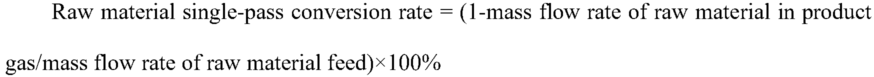 Raw material single-pass conversion rate = (1-mass flow rate of raw material in product gas/mass flow rate of raw material feed)×100% 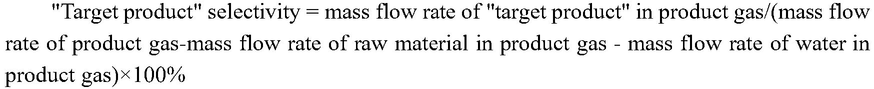 "Target product" selectivity = mass flow rate of "target product" in product gas/(mass flow rate of product gas-mass flow rate of raw material in product gas - mass flow rate of water in product gas)×100%

### Example 1

This embodiment uses the apparatus shown in FIG. 1.

In this embodiment, the raw material is methanol. The catalyst is SAPO-34 molecular sieve catalyst. The process operating conditions of the reaction zone (A) are: gas superficial linear velocity of 7.0m/s, temperature of 450°C, pressure of 50kPa, bed density of 100kg/m³, and reaction contact time of 0.8s. The process operating conditions of the catalyst retention zone (B) are: gas superficial linear velocity of 0.2m/s, temperature of 450°C, and bed density of 500kg/m³. The circulation intensity of catalyst flowing from the catalyst retention zone (B) to the reaction zone (A) is 1000kg/(m²·s).

In Example 1, the single-pass conversion rate of methanol is 99.9%, and the product composition is 50%wt ethylene, 41%wt propylene and 9%wt other components. The other components are methane, ethane, propane, butane, butene, C₅+ hydrocarbons, hydrogen, CO, CO₂ and coke, etc.

### Example 2

This embodiment uses the apparatus shown in FIG. 1.

In this embodiment, the raw material is methanol. The catalyst is SAPO-18 molecular sieve catalyst. The process operating conditions of the reaction zone (A) are: gas superficial linear velocity of 5.0m/s, temperature of 360°C, pressure of 100kPa, bed density of 200kg/m³, and reaction contact time of 1.1s. The process operating conditions of the catalyst retention zone (B) are: gas superficial linear velocity of 0.1m/s, temperature of 360°C, and bed density of 740kg/m³. The circulation intensity of catalyst flowing from the catalyst retention zone (B) to the reaction zone (A) is 860kg/(m²·s)

In Example 2, the single-pass conversion rate of methanol is 99.6%, and the product composition is 38%wt ethylene, 51%wt propylene and 11%wt other components. The other components are methane, ethane, propane, butane, butene, C₅+ hydrocarbons, hydrogen, CO, CO₂ and coke, etc.

### Example 3

This embodiment uses the apparatus shown in FIG. 1.

In this embodiment, the raw material is dimethyl ether. The catalyst is SAPO-34 molecular sieve catalyst. The process operating conditions of the reaction zone (A) are: gas superficial linear velocity of 3.0m/s, temperature of 410°C, pressure of 180kPa, bed density of 270kg/m³, and reaction contact time of 1.8s. The process operating conditions of the catalyst retention zone (B) are: gas superficial linear velocity of 0.08m/s, temperature of 410°C, and bed density of 620kg/m³. The circulation intensity of catalyst flowing from the catalyst retention zone (B) to the reaction zone (A) is 710kg/(m²·s).

In Example 3, the single-pass conversion rate of methanol is 99.8%, and the product composition is 44%wt ethylene, 46%wt propylene and 10%wt other components. The other components are methane, ethane, propane, butane, butene, C₅+ hydrocarbons, hydrogen, CO, CO₂ and coke, etc.

### Example 4

This embodiment uses the apparatus shown in FIG. 1.

In this embodiment, the raw material is mixed C4, containing 34%wt butane, 63%wt butene, 1%wt pentane, and 2%wt pentene. The catalyst is ZSM-5 molecular sieve catalyst. The process operating conditions of the reaction zone (A) are: gas superficial linear velocity of 2.0m/s, temperature of 690°C, pressure of 500kPa, bed density of 390kg/m³, and reaction contact time of 2.7s. The process operating conditions of the catalyst retention zone (B) are: gas superficial linear velocity of 0.05m/s, temperature of 690°C, and bed density of 830kg/m³. The circulation intensity of catalyst flowing from the catalyst retention zone (B) to the reaction zone (A) is 660kg/(m²·s).

In Example 4, the single-pass conversion rate of mixed C4 is 77%, and the product composition is 17%wt ethylene, 65%wt propylene and 18%wt other components. The other components are methane, ethane, propane, butane, butene, C₅+ hydrocarbons, hydrogen, CO, CO₂ and coke, etc.

### Example 5

This embodiment uses the apparatus shown in FIG. 1.

In this embodiment, the raw material is naphtha. The catalyst is ZSM-5 molecular sieve catalyst. The process operating conditions of the reaction zone (A) are: gas superficial linear velocity of 1.5m/s, temperature of 650°C, pressure of 300kPa, bed density of 500kg/m³, and reaction contact time of 3.7s. The process operating conditions of the catalyst retention zone (B) are: gas superficial linear velocity of 0.02m/s, temperature of 650°C, and bed density of 900kg/m³. The circulation intensity of catalyst flowing from the catalyst retention zone (B) to the reaction zone (A) is 500kg/(m²·s).

In Example 5, the single-pass conversion rate of naphtha is 83%, and the product composition is 12%wt ethylene, 56%wt propylene, 19%wt butene and 13%wt other components. The other components are methane, ethane, propane, butane, hydrogen, CO, CO₂ and coke,

The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A high-density fast fluidized bed reactor, **characterized in that** the high-density fast fluidized bed reactor comprises a reactor outer shell, a reactor inner shell, a delivery pipe, a first gas-solid separation device, and a second gas-solid separation device;
wherein the reactor inner shell is located in a lower part of the reactor outer shell;
the delivery pipe is located in a central region of an upper-middle part of the high-density fast fluidized bed reactor;
a region enclosed by the reactor inner shell is a reaction zone;
a bottom end of the delivery pipe is in communication with a top end of the reaction zone; an annular region enclosed by the reactor outer shell and the reactor inner shell is a catalyst retention zone;
a bottom of the reaction zone is in communication with a bottom of the catalyst retention zone;
a region enclosed by the reactor outer shell and the delivery pipe is a gas-solid separation zone;
the catalyst retention zone is in communication with the gas-solid separation zone and is located below the gas-solid separation zone;
the first gas-solid separation device and the second gas-solid separation device are both located in the gas-solid separation zone;
an upper opening of the delivery pipe is connected to an inlet of the first gas-solid separation device;
a catalyst outlet of the first gas-solid separation device is located in a lower part of the gas-solid separation zone.

2. The high-density fast fluidized bed reactor according to claim 1, **characterized in that** an inlet of the second gas-solid separation device is located in the gas-solid separation zone, and a catalyst outlet of the second gas-solid separation device is located in a lower part of the gas-solid separation zone.

3. The high-density fast fluidized bed reactor according to any one of claims 1 to 2, **characterized in that** the first gas-solid separation device and the second gas-solid separation device each independently adopt one or more groups of gas-solid cyclone separators, each group of gas-solid cyclone separators comprising a first-stage gas-solid cyclone separator and a second-stage gas-solid cyclone separator.

4. The high-density fast fluidized bed reactor according to any one of claims 1 to 3, **characterized in that** the high-density fast fluidized bed reactor comprises a reactor gas collection chamber and a product gas delivery pipe;
wherein the reactor gas collection chamber is located at a top of the high-density fast fluidized bed reactor;
the product gas delivery pipe is connected to a top of the reactor gas collection chamber;
a gas outlet of the first gas-solid separation device and a gas outlet of the second gas-solid separation device are both connected to the reactor gas collection chamber.

5. The high-density fast fluidized bed reactor according to any one of claims 1 to 4, **characterized in that** the high-density fast fluidized bed reactor comprises a catalyst distribution pipe, a fluidizing steam distributor, a raw material distributor, a catalyst withdrawal pipe, and a catalyst input pipe;
wherein the catalyst distribution pipe passes through the reactor inner shell to communicate the catalyst retention zone and the reaction zone;
the fluidizing steam distributor is located at a bottom of the catalyst retention zone;
the raw material distributor is located at a bottom of the reaction zone;
the catalyst withdrawal pipe passes through the reactor outer shell and is located in a lower part of the catalyst retention zone;
the catalyst input pipe passes through the reactor outer shell and is located in a lower part of the gas-solid separation zone.

6. The high-density fast fluidized bed reactor according to any one of claims 1 to 5, **characterized in that** a lower surface of the catalyst distribution pipe has openings.

7. A use method of the high-density fast fluidized bed reactor according to any one of claims 1 to 6, **characterized in that** the method comprises:
(S-1) catalyst enters the gas-solid separation zone through the catalyst input pipe, and then enters the catalyst retention zone;
(S-2) vaporized raw material enters the reaction zone from the raw material distributor, contacts the catalyst, and generates product gas; the product gas carries the catalyst through the delivery pipe into the first gas-solid separation device; after gas-solid separation, the catalyst enters the catalyst retention zone;
(S-3) steam enters the catalyst retention zone from the fluidizing steam distributor; the steam carries a small amount of catalyst from the catalyst retention zone into the second gas-solid separation device in the gas-solid separation zone; after gas-solid separation, the catalyst returns to the catalyst retention zone;
(S-4) catalyst in the catalyst retention zone enters the reaction zone through the catalyst distribution pipe; catalyst in the catalyst retention zone enters the bottom of the reaction zone through the bottom of the catalyst retention zone;
(S-5) catalyst in the catalyst retention zone is discharged through the catalyst withdrawal pipe.

8. The method according to claim 7, **characterized in that** the product gas in step (S-2) and the steam in step (S-3) enter the reactor gas collection chamber and enter the downstream process section through the product gas delivery pipe.

9. The method according to any one of claims 7 or 8, **characterized in that** the process operating conditions of the reaction zone are: gas superficial linear velocity of 1.5-7.0m/s, temperature of 350-700°C, pressure of 50-500kPa, bed density of 100-500kg/m³, and reaction contact time of 0.5~4s.

10. The method according to any one of claims 7 to 9, **characterized in that** the process operating conditions of the catalyst retention zone are: gas superficial linear velocity of 0.02-0.2m/s, temperature of 350-700°C, and bed density of 500-900kg/m³.

11. The method according to any one of claims 7 to 10, **characterized in that** the circulation intensity of catalyst flowing from the catalyst retention zone to the reaction zone is 500-1000kg/(m²·s).

12. The method according to any one of claims 7 to 11, **characterized in that** the product gas is a product gas containing olefins.

13. The method according to any one of claims 7 to 12, **characterized in that** the raw material comprises methanol and/or dimethyl ether.

14. The method according to any one of claims 7 to 13, **characterized in that** the catalyst is a SAPO molecular sieve.

15. The method according to any one of claims 7 to 14, **characterized in that** the raw material comprises C₃+ hydrocarbons.

16. The method according to any one of claims 7 to 15, **characterized in that** the catalyst is ZSM-5 molecular sieve.
